(19) Europäisches Patentamt
European Patent Office
Office européen des brevets

(11) Publication number: 0 066 440
B1

(12) **EUROPEAN PATENT SPECIFICATION**

(45) Date of publication of patent specification: 31.07.85

(51) Int. Cl.⁴: C 07 D 405/12

(21) Application number: 82302659.6

(22) Date of filing: 25.05.82

(54) **Chemical process.**

(30) Priority: 27.05.81 GB 8116155

(43) Date of publication of application: 08.12.82 Bulletin 82/49

(45) Publication of the grant of the patent: 31.07.85 Bulletin 85/31

(84) Designated Contracting States: AT BE CH DE FR GB IT LI LU NL SE

(58) References cited:
EP-A-0 003 677
EP-A-0 004 793
EP-A-0 017 679
EP-A-0 049 173

(73) Proprietor: SMITHKLINE BECKMAN CORPORATION
P.O. Box 7929 1 Franklin Plaza
Philadelphia Pennsylvania 19101 (US)

(72) Inventor: Labaw, Clifford Steven
230 Penlyn Pike
Penlyn Pennsylvania 19477 (US)
Inventor: Mendelson, Wilford Lee
592 General Learned Road
King of Prussia Pennsylvania 19406 (US)
Inventor: Tremper, Alan William
329 Owen Avenue
Lansdowne Pennsylvania 19026 (US)

(74) Representative: Waters, David Martin, Dr. et al
Smith Kline & French Laboratories Ltd. Patent Department Mundells
Welwyn Garden City Hertfordshire AL7 1EY (GB)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 066 440 B1

Courier Press, Leamington Spa, England.

**Description**

This invention provides a process for preparing 2-[2-[(2-furylmethyl)thio]ethylamino]-5-[(6-methyl-3-pyridinyl)methyl]-4(1*H*)-pyramidinone. This compound is disclosed in European Patent Specification 3677 and is particularly useful as an intermediate to prepare the corresponding 5-dimethylaminoethyl compound which has potent activity as a histamine $H_2$-antagonist.

EP-A-17679 discloses the ring closure of a compound of the formula (II) as shown below and nitroguanidine.

The process of this invention is represented by the following reaction sequence:

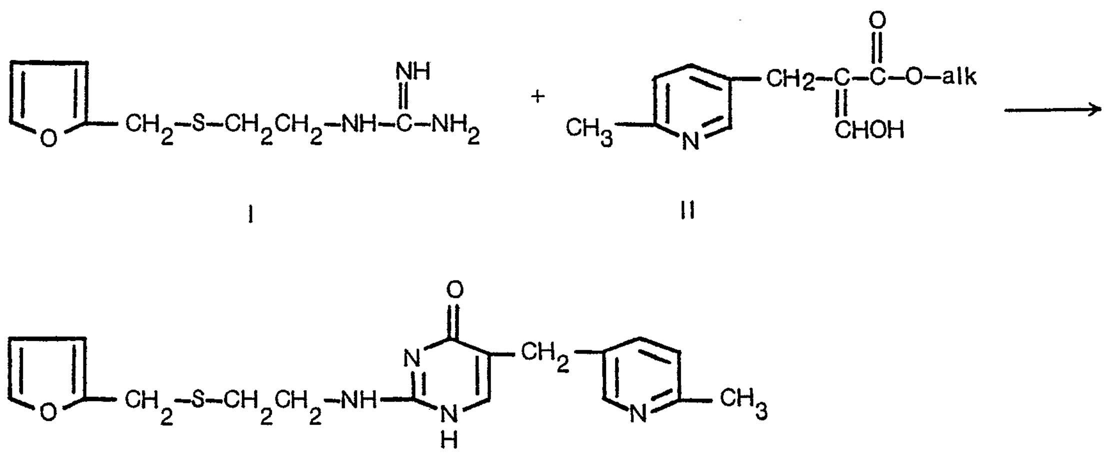

I

II

III

In this reaction sequence, alk is a lower alkyl of 1-6 carbons, usually methyl or ethyl.

According to the process of this invention substantially equimolar quantities of 2-[(2-furylmethyl)thio]ethylguanidine (I), conveniently generated *in situ* from one of its acid addition salts, and a lower alkyl 2-formyl-3-(6-methyl-3-pyridinyl)propionate (II) are reacted in the presence of a base, for example, an alkali metal hydroxide, carbonate or lower alkoxide. Sodium or potassium methoxides, ethoxides, n-propoxides or isopropoxides are most useful.

The reaction is carried out in a solvent toward which the reactants are chemically inert and in which the reactants are soluble enough to react, for example, an alcoholic solvent of from 1—5 carbons such as methanol, ehtanol, n-propanol, n-butanol or isopropanol, an aromatic solvent such as benzene, toluene or xylene, dimethylformamide, dimethylacetamide, dimethylsulfoxide, water, an ester solvent such as ethyl acetate or a halohydrocarbon solvent such as methylene chloride or chloroform. Preferably n-propanol or toluene are used. The solvents may also be used in various combinations.

Preferably, the process is carried out using a sodium or potassium alkoxide in a lower alkanol solvent.

If an acid addition salt of the guanidine (I) is used, at least one mole of a base is needed to generate the basic compound. For the condensation itself, less than one molar equivalent of a base, for example 1/4 molar equivalent, is satisfactory; however, an excess of base can be used. The rection is allowed to proceed at from ambient up to reflux temperature until complete. Most conveniently the reaction described above is carried out in n-propanol at reflux temperature for from about 1—5 hours.

When the reaction is complete, the volume of solvent is reduced. Water is added to the reaction mixture followed by cooling to separate the desired pyrimidinone (III). Unexpectedly, the condensation is selective and the desired product, i.e. the pyrimidinone of Formula III, is produced as a large proportion of the product of reaction with only a small proportion of the alternative isomeric condensation products (1- and 3-substituted-2-aminopyrimidinones), the ratio being about 9 to 10:1 of the desired product. The product can be purified as known to the art but preferably as described in the following example to give from 60—85% yields of isolated pure product. Alternatively, the impure product may be used as such or *in situ* to prepare the 5-dimethylaminoethyl derivative.

The 5-dimethylaminomethyl derivative is prepared from 2-[[2-[(2-furylmethyl)thio]ethyl]amino]-5-[(6-methyl-3-pyridinyl)methyl]-4(1H)-pyrimidinone (III) by reaction with a Mannich reagent to insert a dimethylaminoethyl group at the 5-position of the furyl ring by procedures described in European Patent Specification 3677.

There are two unexpected features of the chemical process of this invention which are particularly advantageous in obtaining good purity of the products.

The first is that this process, to form a specifically substituted pyrimidinone ring, has been found to be highly selective with only about 10% of the undesired isomers formed. The second concerns the purification of the furylpyrimidinone III to remove the unwanted isomers. Slurrying of the crude reaction product in an organic solvent for example a low molecular weight ester, ketone or ether, such as a lower

alkyl acetate, methyl ethyl ketone, acetone or tetrahydrofuran, especially ethyl acetate or acetone, is very effective in removing the isomeric byproducts which are selectively more soluble in said solvents. This method of purification is surprisingly efficient considering the close structural relationship between the desired product and the alternative isomeric condensation products.

The following example illustrates the process of this invention.

Example 1

A mixture of 1600 g (3.88 m) of 2-[(2-furylmethyl)thio]ethylamine hemisulfate and 2.8 L of n-propanol was heated to 50° and 400 ml of 50% aqueous cyanamide added thereto. The pH was adjusted to 7.9 with 10N sodium hydroxide solution. The reaction mixture was heated to reflux. Additional 400 ml portions of aqueous cyanamide were added at 1 hour, 2 hours and 4 hours maintaining the pH at 8.3 ± .2 using 10N sodium hydroxide and 6N sulfuric acid. After 7.5 hours at reflux, the reaction was diluted again with 3.8L of isopropanol then filtered hot. The filtrate was diluted again with 6L of isopropanol (40—43°) and stirred for 16 hours as it cooled. Cooling to 5—8° gives 1528 g (79%) of 2-[(2-furylmethyl)thio]ethylguanidine hemisulfate.

A mixture of 992 g of the guanidine sulfate, 6L of n-propanol and 880 ml of 25% sodium methoxide in methanol was heated to 50°. Ethyl 2-formyl-3-(6-methyl-3-pyridinyl)propionate (91 g) was added and the mixture heated to reflux at which time five 157 g portions of the formyl compound were added at half hour time intervals [total 876 g (4.0 ml)]. After 3 hours, about 2L of solvent was removed by distillation. Water (14L) was added to the hot solution. The resulting slurry was allowed to stir for 12 hours cooing to room temperature then was cooled to 5—8°. The solid product was separated, dried, slurried in boiling anhydrous ethyl acetate and dried again to give 897 g (63%) 2-[[2-[(2-furylmethyl)thio]ethylamino]-5-[(6-methyl-3-pyridinyl)methyl]-4-(1*H*)-pyrimidinone (III).

The ethyl acetate slurry process effectively removes the isomeric byproducts. Other organic solvents such as acetone may also be used in this purification procedure.

**Claims**

1. The method of preparing a furyl pyrimidinone compound of the formula:

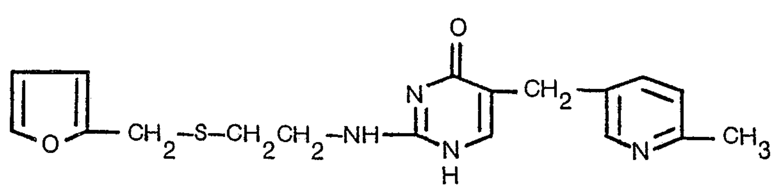

which comprises reacting in substantially equimolar quantities a guanidine compound of the formula:

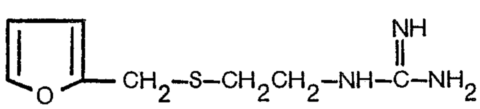

and a 2-formyl-3-pyridinylpropionate compound of the formula:

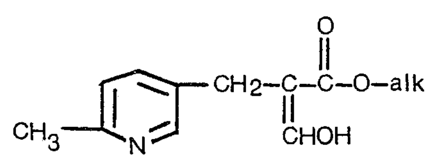

in which alk is lower alkyl of 1—6 carbon atoms, in the presence of a base in an organic solvent toward which the reactants are chemically inert and in which the reactants are soluble enough to react.

2. The process of claim 1 in which alk is methyl or ethyl, the solvent for reaction is n-propanol or toluene and the base is sodium or potassium methoxide, ethoxide, n-propoxide or isopropoxide.

3. The process of claims 1 or 2 in which the furyl pyrimidinone is purified by slurrying in ethyl acetate, acetone or a similar organic solvent.

**Patentansprüche**

1. Verfahren zur Herstellung einer Furyl-pyrimidinon-Verbindung der Formel:

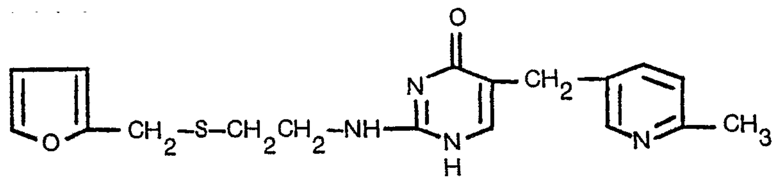

gekennzeichnet durch Umsetzen von im wesentlichen äquimolaren Mengen einer Guanidinverbindung der Formel:

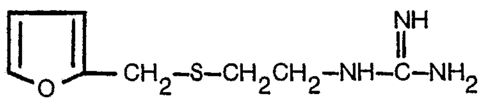

und eines 2-Formyl-3-pyridinylpropionsäureesters der Formel:

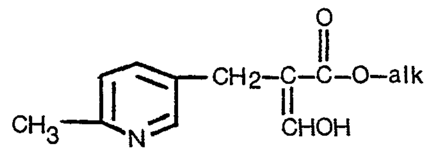

in der alk einen Nideralkylrest mit 1 bis 6 Kohlenstoffatomen bedeutet, in Gegenwart einer Base in einem organischen Lösungsmittel, dem gegenüber die Reaktionsteilnehmer chemisch inert sind und in dem die Reaktionsteilnehmer ausreichend löslich sind um miteinander zu reagieren.

2. Verfahren nach Anspruch 1, wobei der Rest alk eine Methyl- oder Äthylgruppe bedeutet, das Lösungsmittel für die Umsetzung n-Propanol oder Toluol ist und die Base Natrium- oder Kaliummethoxid, -äthoxid, n-Propoxid oder Isopropoxid ist.

3. Verfahren nach Anspruch 1 oder 2, wobei das Furylpyrimidinon durch Aufschlemmung in Äthylacetat, Aceton oder einem ähnlichen organischen Lösungsmittel gereinigt wird.

**Revendications**

1. La méthode de préparation d'un composé de pyrimidinone furyle de la formule:

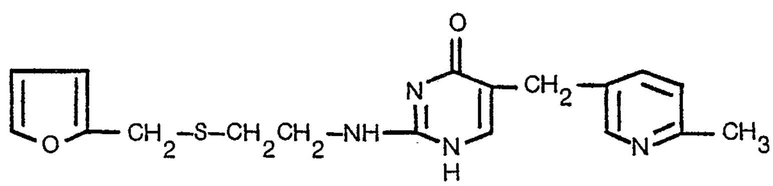

qui comprend la mise en réaction, en quantités substantiellement équimoléculaires d'un composé guanidine de la formule:

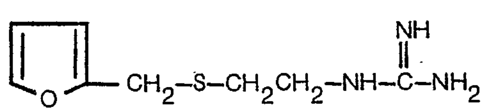

et d'un composé 2-formyle-3-pyridinylpropionate de la formule:

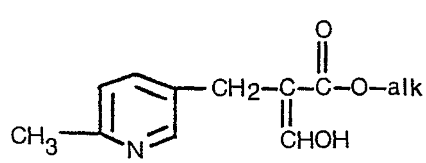

dans laquelle alk est de l'acoyle plus faible ayant 1—6 atomes de carbone, en présence d'une base dans un

solvant organique envers lequel les réactants sont chimiquement inactifs et dans lequel les réactants sont assez solubles pour réagir.

2. Le procédé de la revendication 1 dans lequel alk est méthyle ou éthyle, le solvant pour la réaction est n-propanol ou toluène et la base est du méthoxyde de potassium ou de sodium, de l'éthoxyde, du n-propoxyde ou de l'isopropoxyde.

3. Le procédé des revendications 1 ou 2 dans lequel le furyle pyrimidinone est purifié en étant réduit en boue dans de l'éthyle acétate, de l'acétone ou un solvant organique similaire.